# EUROPEAN PATENT SPECIFICATION

(11) **EP 0 812 599 B1**
(45) Date of publication and mention of the grant of the patent: **07.08.2002**
(21) Application number: 97114538.8
(22) Date of filing: 06.05.1992
(51) Int. Cl.: A61M 25/01, A61M 25/09

(54) **Catheter guide wire**
Katheterführungsdraht
Fil de guidage pour cathéter

(30) Priority: 07.05.1991 US 696585
(43) Date of publication of application: 17.12.1997
(62) Divisional of application: 92912258.8
(73) Proprietor: TARGET THERAPEUTICS, INC., Fremont, CA 94538 (US)
(72) Inventor: Sepetka, Ivan, Redwood City, California 94063 (US)
(74) Representative: Price, Nigel John King

(56) References cited:
- EP-A- 0 274 412
- EP-A- 0 386 921
- EP-A- 0 405 823
- US-A- 4 721 117
- US-A- 4 884 579

## Description

### Technical Field

This invention is in the general field of surgical instruments and relates specifically to guide wires that are used in cardiovascular and endovascular procedures to facilitate the placement of catheters within the vasculature of patients.

### Background

The general procedure for placing catheters within vessels is to track a guide wire through the vessel to the desired position and advance the catheter over the guide wire. Guide wires are required because the catheters themselves do not have sufficient column strength or torsional strength to be able to be tracked or steered through the vessel. See, for instance, U.S. Patent No. 4,884,579.

Several types of guide wires for use in catheter placement have been proposed. The simplest type of wire has a preferred diameter of between about 0.20-1.0 mm. The distal end of the wire may be provided with a bent tip which can be oriented, by means of a guide structure at the proximal end, to guide the wire along a selected vascular path. Ideally, torque transmission should be controlled, such that a selected wire rotation at the wire's proximal end produces a corresponding rotation of the distal end. Further, radiopacity is desired such that a physician may see over the entire vasculature accessed by the guide wire.

US-A-4,884,579 discloses a catheter guide wire with three sections with progressively greater flexibility and sliding properties: 1. a semi-rigid, torqueable proximal wire section that is between about 50-250 cm in length, formed of a proximal wire core segment having an outer diameter of between about 0.25-1.0 mm; 2. a more flexible intermediate section that has a length between about 20-60 cm and is formed from an intermediate wire-core segment having a reduced diameter of between about 0.10-0.50 mm, and a low-friction, flexible polymer tube covering which encases the intermediate core segment; and 3. a most distal end section with a length between about 1-10 cm and formed from a distal wire core segment having a reduced diameter of between about 0.05-0.15 mm, and a flexible sleeve covering the distal end segment and providing column strength thereto.

US-A-4 721 117 discloses a torsionally stabilised guidewire, the core wire of which has three sections of different diameters. A helical stainless steel coil extends from a midpoint of the proximal section of the core wire to the distal tip of the core wire. The wire forming the helical coil is covered with Teflon.

The guidewire disclosed in EP-A-0 274 412 includes a core wire, an outer helical coil and an inner ribbon coil which is more resistant to stretching out than round cross-sectional wire. The outer helical coil extends to the distal tip of the guidewire.

According to the present invention there is provided a guidewire for use within a patient's vasculature, comprising in combination:
(a) a semi-rigid, torqueable proximal wire section,
(b) a more flexible intermediate section comprising an intermediate wire core segment of reduced diameter surrounded by a radioopaque ribbon coil, and a flexible polymer tube covering which encases the ribbon coil, to improve torque transmission along the guidewire, to increase radioopacity of the intermediate section over that of the bare intermediate wire core segment without substantially increasing its stiffness, and to provide a low friction surface and column support to the reduced diameter intermediate wire core segment; and
(c) a most flexible distal end section formed from a distal wire core segment with a helical coil, separate from said radioopaque ribbon coil, covering the distal end segment and providing column strength thereto,
wherein said helical coil provides for a frictional coefficient of the distal end section that is greater than the frictional coefficient of the intermediate section.

Embodiments of guidewire constructed in accordance with the present invention will now be described, by way of example only, with reference to the accompanying drawings, described below.
Fig. 1 shows fragmentary portions of a guide wire constructed according to one embodiment of the invention;
Fig. 2 shows fragmentary portions of a guide wire constructed according to another embodiment of the invention.

Like parts are referred to by the same reference numerals in the Figures.

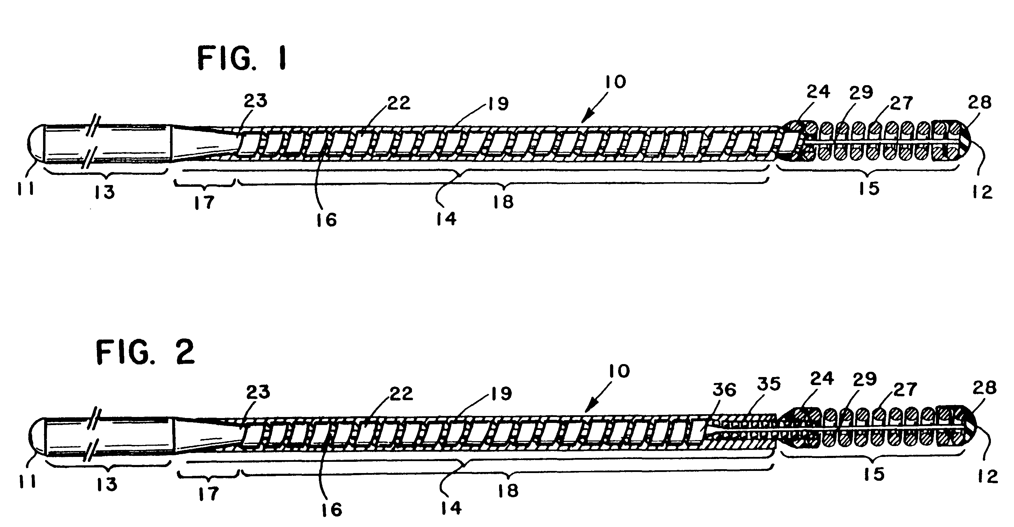

Fig. 1 shows a guide wire generally designated 10, constructed according to one embodiment of the invention. The wire is a flexible torqueable wire having an overall length of about 70-300 cm between its proximal and distal ends 11 and 12, respectively, and a maximum outer diameter of between about 0.20-1.0 mm. The major portion of the wire is a flexible proximal section 13 whose overall length ranges from about 50-250 cm. This section is followed by a more flexible intermediate section 14 having a length between about 20-60 cm and a most flexible distal end section 15 whose length is between about 1-10 cm.

A wire core 16 in the guide wire 10 is formed of a flexible, torqueable wire filament material, such as stainless steel. The diameter of the wire core, at its maximum, is between about 0.20-1.0 mm. The segment of the core forming proximal section 13 of guide wire 10 has a substantially uniform diameter along its length, and corresponds to the maximum diameter of the core, i.e., between 0.20-1.0 mm.

Within the intermediate section 14 of the wire, the core is tapered from the proximal-section diameter down to a reduced diameter which is preferably about 0.10-0.50 mm and between about 10%-50% of the diameter of the core's proximal segment 13. Thus, for example, where the proximal section core diameter is 0.46 mm, the core tapers to a minimum of between about 0.05-0.23 mm. The length of tapered segment 17 is typically between about 10%-50% that of reduced-diameter segment 18, and the two segments together make up the length of the intermediate wire section 14, i.e., about 20-60 cm.

The wire core 16 of intermediate section 14 is covered along its length by a flexible polymer covering 19. The major function of covering 19 is to provide a low-friction surface along intermediate section 14, and more particularly, a surface which has less friction than the surface of adjacent distal segment 15 and proximal segment 13 (the wire core itself). Covering 19 preferably also functions to provide column support to the reduced-diameter core of the intermediate section, 18, to reduce the tendency of this section to buckle under axial compression.

Covering 19 is preferably formed of a polymer, such as TEFLON™, polyolefin, or polyurethane which can be bonded or otherwise tightly affixed to the core wire, and which itself has a low-friction surface, or can be coated with a low-friction surface. Other suitable coverings include a tube formed from virtually any polymer having exposed hydrogens, such as polyester, polyolefins, polycarbonate, polyvinylchloride, latex or silicon rubber, polystyrene, and polyacrylics, and a surface coating formed of a highly hydrophilic, low-friction polymer, such as polyvinylpyrrolidone (PVP), polyethyleneoxide, or polyhydroxyethylmethacrylate (polyHEMA) or copolymers thereof.

Beneath polymer covering 19, a ribbon of radiopaque metal 22, such as platinum, gold, tungsten, or their alloys is wound around the wire core 16. As shown, the ribbon coil 22 extends from tapered segment 17 of intermediate section 14 at junction 23, to the distal junction 24 of intermediate section 14. The ribbon coil 22 has a thickness of about 0.015 to 0.050 mm, preferably about 0.025 mm and a width of about 0.050 to 0.130 mm, preferably about 0.075 mm. There are approximately 5 to 15 complete turns of ribbon per millimeter of wire core, and preferably about 10 complete turns of ribbon per millimeter of wire core.

The distal section 15 of guide wire 10 is fully or partially encased in flexible sleeve 27. Sleeve 27 shown in Fig. 1 is a soft, flexible helical coil which is formed conventionally, e.g., as a winding of radiopaque wire strand, such as platinum, gold, or tungsten strand. The wire strand has a diameter of about 0.050 to 0.100 mm and preferably about 0.075 mm. As shown, sleeve 27 extends from junction 24 to distal end 12 of guide wire 10. Attachment of the sleeve 27 to wire core 16 is preferably by two or three solder or weld joints, one at proximal junction 24 and a second at rounded distal junction 28.

In addition to providing a mechanism for wire bending near wire tip 12, sleeve 27 also gives distal section 15 of guide wire 10 increased column strength (in the axial direction), and reduces the chance of buckling in this section with axial compression. At the same time, the combined flexibility of reduced diameter core 29 and sleeve 27 are compatible with a series of sharp bends, as the wire is moved through a patient's vasculature. Rounded joint 28 at the end of guide wire 10 acts to shield vessel walls from the sharp end of wire core 16. Further, the distal section of the wire, 15, with the associated sleeve 27, provides the section with a higher frictional coefficient than that of the adjacent intermediate section, 14. The higher-friction surface in this distal section, 15, functions specifically, during a catheter placement operation, to help anchor distal section 15 against a vessel wall at a vessel junction.

Distal wire core 29 has a substantially uniform cross-section. The core may be cylindrical with diameter of between 0.05 and 0.15 mm or flattened with a rectangular cross-section dimensioned 0.025 mm by 0.075 mm. The wire core has a tapered section at junction 24 that covers between about 10-50% of the core's distal segment.

Fig. 2 is another embodiment of the invention that is essentially the same as Fig. 1 except for the replacement of a portion of helical ribbon coil 22 with inner wire coil 35. At the distal end of helical ribbon coil 22, there is a soft flexible helical coil 24 which is formed conventionally, e.g., as a winding of radiopaque wire strand, such as platinum, gold or tungsten. As shown, coil 35 extends from helical ribbon coil 22 at junction 36 to the proximal end of distal segment 15 at junction 24. This inner coil 35 serves as an anchor point for the distal end of flexible helical coil 22 and also as an anchor point for the polymer covering 19 on intermediate core section 14.

## Claims

1. A guidewire (10) for use within a patient's vasculature, comprising in combination:
(a) a semi-rigid, torqueable proximal wire section (13),
(b) a more flexible intermediate section (14) comprising an intermediate wire core segment (16) of reduced diameter surrounded by a radioopaque ribbon coil (22), and a flexible polymer tube covering (19) which encases the ribbon coil (22), to improve torque transmission along the guidewire, to increase radioopacity of the intermediate section (14) over that of the bare intermediate wire core segment (16) without substantially increasing its stiffness, and to provide a low friction surface and column support to the reduced diameter intermediate wire core segment (16); and
(c) a most flexible distal end section (15) formed from a distal wire core segment (29) with a helical coil (27), separate from said radioopaque ribbon coil, covering the distal end segment and providing column strength thereto,
wherein said helical coil (27) provides for a frictional coefficient of the distal end section (15) that is greater than the frictional coefficient of the intermediate section (14).

2. The guidewire of claim 1, wherein the ribbon coil (22) is formed from a radioopaque material selected from the group consisting of platinum, gold, tungsten and their alloys.

3. The guidewire of claim 1 or claim 2, wherein:
(a) the proximal wire section (13) has an outer diameter between about 0.25-1.00 mm,
(b) the intermediate wire core segment (16) has a reduced diameter of between about 0.10-0.50 mm, and
(c) the distal wire core segment has a further reduced cross-sectional area.

4. The guidewire of claim 3, wherein the distal wire core segment (29) is cylindrical and has a diameter of between about 0.05-0.15 mm.

5. The guidewire of claim 3, wherein the distal wire core segment (29) is rectangular with cross-sectional dimensions of 0.025 by 0.075 mm.

6. The guidewire of claim 1 or claim 2, wherein:
(a) the proximal wire section (13) has an outer diameter of between about 0.25-0.50 mm,
(b) the intermediate wire core segment (16) has a reduced diameter of between about 0.10-0.20 mm, and
(c) the distal wire core segment has a further reduced diameter of between about 0.05-0.13 mm.

7. The guidewire of any one of the preceding claims, wherein a platinum helical coil (35) wrapped around the wire core at the proximal end of the distal section (15) serves as an anchor point for the proximal end of the distal helical coil (27) and the anchor point for the distal end of the flexible polymer covering (19).

## Patentansprüche

1. Führungsdraht (10) zur Verwendung in dem Gefäßsystem eines Patienten, welcher in Kombination umfasst:
(a) einen halbstarren, verdrehbaren proximalen Drahtabschnitt (13),
(b) einen biegsameren Zwischenabschnitt (14), welcher ein von einer strahlenundurchlässigen Bandspule (22) umgebenes dazwischenliegendes Drahtkernsegment (16) mit verringertem Durchmesser und eine biegsame Polymerschlauchumhüllung (19), welche die Bandspule (22) umhüllt, umfasst, um die Drehmomentübertragung entlang des Führungsdrahts zu verbessern, die Strahlenundurchlässigkeit des Zwischenabschnitts (14) verglichen mit der des blanken dazwischenliegenden Drahtkernsegments (16) zu erhöhen, ohne seine Steifigkeit erheblich zu erhöhen, und dem dazwischenliegenden Drahtkernsegment (16) mit verringertem Durchmesser eine reibungsarme Oberfläche und Knickfestigkeit zu verleihen, sowie
(c) einen aus einem distalen Drahtkernsegment (29) ausgebildeten, sehr biegsamen distalen Endabschnitt (15) mit einer von der strahlenundurchlässigen Bandspule getrennten spiralförmigen Spule (27), welche das distale Endsegment umhüllt und diesem Knickfestigkeit verleiht,
**dadurch gekennzeichnet, dass** die spiralförmige Spule (27) einen Reibungskoeffizienten des distalen Endabschnitts (15) ermöglicht, der größer als der Reibungskoeffizient des Zwischenabschnitts (14) ist.

2. Führungsdraht nach Anspruch 1, **dadurch gekennzeichnet, dass** die Bandspule (22) aus einem strahlenundurchlässigen Material gewählt aus der Gruppe bestehend aus Platin, Gold, Wolfram und deren Legierungen gebildet ist.

3. Führungsdraht nach Anspruch 1 oder 2, **dadurch gekennzeichnet, dass**:
(a) der proximale Drahtabschnitt (13) einen Außendurchmesser von etwa 0,25 bis 1,00 mm aufweist,
(b) das dazwischenliegende Drahtkernsegment (16) einen verringerten Durchmesser von etwa 0,10 - 0,50 mm aufweist und
(c) das distale Drahtkernsegment eine weiter verringerte Querschnittfläche aufweist.

4. Führungsdraht nach Anspruch 3, **dadurch gekennzeichnet, dass** das distale Drahtkernsegment (29) zylindrisch ist und einen Durchmesser von etwa 0,05 - 0,15 mm aufweist.

5. Führungsdraht nach Anspruch 3, **dadurch gekennzeichnet, dass** das distale Drahtkernsegment (29) rechteckig ist, mit Querschnittmaßen von 0,025 auf 0,075 mm.

6. Führungsdraht nach Anspruch 1 oder 2, **dadurch gekennzeichnet, dass**:
(a) der proximale Drahtabschnitt (13) einen Außendurchmesser von etwa 0,25 - 0,50 mm aufweist,
(b) das dazwischenliegende Drahtkernsegment (16) einen verringerten Durchmesser von etwa 0,10 - 0,20 mm aufweist und
(c) das distale Drahtkernsegment einen weiter verringerten Durchmesser von etwa 0,05 - 0,13 mm aufweist.

7. Führungsdraht nach einem der vorstehenden Ansprüche, **dadurch gekennzeichnet, dass** eine an dem proximalen Ende des distalen Abschnitts (15) um den Drahtkern gewickelte spiralförmige Platinspule (35) als Ankerpunkt für das proximale Ende der distalen spiralförmigen Spule (27) und Ankerpunkt für das distale Ende der biegsamen Polymerumhüllung (19) dient.

## Revendications

1. Fil de guidage (10) destiné à être utilisé à l'intérieur du système vasculaire d'un patient, comprenant, en combinaison :
(a) une section de fil proximale semi-rigide pouvant subir un couple (13),
(b) une section intermédiaire plus souple (14) comprenant un segment de coeur de fil intermédiaire (16) de diamètre réduit entouré par un serpentin en ruban radio-opaque (22), et un revêtement en tube polymère souple (19) qui renferme le serpentin en ruban (22), de façon à améliorer la transmission de couple le long du fil de guidage, de façon à accroître la radio-opacité de la section intermédiaire (14) par rapport à celle du segment de coeur de fil intermédiaire nu (16) sans accroître sensiblement sa rigidité, et à procurer une surface de faible frottement et un support de colonne au segment de coeur de fil intermédiaire de diamètre réduit (16) ; et
(c) une section d'extrémité distale plus souple (15) constituée par un segment de coeur de fil distal (29) avec un serpentin hélicoïdal (27), séparé dudit serpentin en ruban radio-opaque, recouvrant le segment d'extrémité distale et communiquant une résistance à la flexion par compression axiale à celui-ci,
dans lequel ledit serpentin hélicoïdal (27) produit un coefficient de frottement de la section d'extrémité distale (15) qui est supérieur au coefficient de frottement de la section intermédiaire (14).

2. Fil de guidage selon la revendication 1, dans lequel le serpentin en ruban (22) est formé en un matériau radio-opaque sélectionné parmi le groupe comprenant le platine, l'or, le tungstène et leurs alliages.

3. Fil de guidage selon la revendication 1 ou la revendication 2, dans lequel :
(a) la section de fil proximale (13) a un diamètre extérieur compris entre environ 0,25 et 1,0 mm,
(b) le segment de coeur de fil intermédiaire (16) a un diamètre réduit compris entre environ 0,10 et 0,50 mm, et
(c) le segment de coeur de fil distal a une surface de section transversale encore davantage réduite.

4. Fil de guidage selon la revendication 3, dans lequel le segment de coeur de fil distal (29) est cylindrique et a un diamètre compris entre environ 0,05 et 0,15 mm.

5. Fil de guidage selon la revendication 3, dans lequel le segment de coeur de fil distal (29) est rectangulaire, avec des dimensions de section transversale de 0,025 sur 0,075 mm.

6. Fil de guidage selon la revendication 1 ou la revendication 2, dans lequel :
(a) la section de fil proximale (13) a un diamètre extérieur compris entre environ 0,25 et 0,50 mm,
(b) le segment de coeur de fil intermédiaire (16) a un diamètre réduit compris entre environ 0,10 et 0,20 mm, et
(c) le segment de coeur de fil distal a un diamètre encore davantage réduit, compris entre environ 0,05 et 0,13 mm.

7. Fil de guidage selon l'une quelconque des revendications précédentes, dans lequel un serpentin hélicoïdal en platine (35) enroulé autour du fil de coeur à l'extrémité proximale de la section distale (15) joue le rôle de point d'ancrage pour l'extrémité proximale du serpentin hélicoïdal distal (27) et de point d'ancrage pour l'extrémité distale du revêtement polymère souple (19).
